# EUROPÄISCHE PATENTANMELDUNG

(11) **EP 0 774 261 A2**
(43) Veröffentlichungstag der Anmeldung: **21.05.1997**
(21) Anmeldenummer: 96117930.6
(22) Anmeldetag: 08.11.1996
(51) Int. Cl.: A61K 38/37, C07K 14/755

(54) **Verwendung von von Willebrand-Faktor und pharmazeutische Zubereitung**

(30) Priorität: 10.11.1995 DE 19542004; 25.04.1996 DE 19616540
(71) Anmelder: IMMUNO AG, 1221 Wien (AT)
(72) Erfinder: Schwarz, Hans-Peter, Doz., 1180 Wien (AT); Turecek, Peter, Dr., 3400 Klosterneuburg (AT); Eibl, Johann, Dr., 1180 Wien (AT)
(74) Vertreter: Kolb, Helga, Dr. Dipl.-Chem.

(57) **Zusammenfassung**

Die Erfindung betrifft die Verwendung eines von Willebrand Faktor (vWF) mit einer verlängerten biologischen Halbwertszeit zur Herstellung einer Präparation zur Stabilisierung von Blutgerinnungsfaktor VIII (FVIII:C) in einem Säugetier. Ausserdem wird eine pharmazeutische Präparation zur Verfügung gestellt, enthaltend einen biologisch aktiven FVIII:C/vWF-Komplex mit verbesserten pharmakokinetischen Eigenschaften, welcher Komplex einen vWF mit einer verlängerten biologischen Halbwertszeit enthält.

## Beschreibung

Die Erfindung betrifft eine pharmazeutische Präparation enthaltend einen biologisch aktiven FVIII:C/vWF-Komplex sowie eine neue Verwendung eines von Willebrand Faktor Präparates und ein diagnostisches Set zur Bestimmung eines Defektes der Hämostase in einem Patienten.

Der vWF liegt in Humanplasma als heterogenes Homomultimere vor, mit einem Molekulargewicht von 450 kD bis zu mehr als 10.000 kD. Der vWF dient als Trägerprotein für den Gerinnungsfaktor VIII und trägt zur Adhäsion von Blutplättchen an das Endothel von verletzten Blutgefässen bei.

Bei der Hämophilie (Bluterkrankheit) ist die Blutgerinnung durch Mangel an bestimmten plasmatischen Blutgerinnungsfaktoren gestört. Bei der Hämophilie A beruht die Blutungsneigung auf einem Mangel an Faktor VIII bzw. einem Mangel an vWF. Die Behandlung der Hämophilie A erfolgt durch Ersatz des fehlenden Gerinnungsfaktors durch Faktorenkonzentrate aus Blutkonserven, z.B. durch intravenöse Infusion von Faktor VIII, eines vWF/Faktor VIII-Komplexes oder von vWF.

Es gibt mehrere Krankheitsbilder, die auf Unter- oder Überproduktion des von Willebrand-Faktors zurückzuführen sind. So führt z.B. eine Überproduktion von vWF zur vermehrten Neigung von Thrombosen, wohingegen eine Unterversorgung mit vWF eine vermehrte Blutungsneigung oder verlängerte Blutungszeit zur Folge hat.

Das von Willebrand-Syndrom kann sich in mehreren Erscheinungsformen manifestieren. Alle Formen zeichnen sich durch eine verlängerte Blutungszeit aus, die durch ein absolutes Fehlen eines funktionellen vWF begründet sein können. Der Mangel an vWF kann auch eine phänotypische Hämophilie A hervorrufen, da der vWF ein wesentlicher Bestandteil des funktionellen Faktor VIII-Komplexex ist. In diesen Fällen ist die Halbwertszeit des Faktor VIII derart verringert, dass er seine spezielle Funktionen in der Blutgerinnung nicht wahrnehmen kann.

Patienten mit vW-Krankheit weisen einen Mangel an funktionell aktiven vWF auf, als Ursache für Blutungen. Zur Therapie der vW-Krankheit werden vor allem therapeutische Plasmakonzentrate vorgeschlagen, die den vWF enthalten. Der von Plasma stammende vWF (pdvWF) weist jedoch eine veränderte Multimerenverteilung auf. Die hochmolekularen Multimeren werden während des Herstellungsprozesses abgebaut, weshalb eine verringerte hämostatische Wirkung des Plasmakonzentrats vermutet wird. Für den Abbau der hochmolekularen vWF-Multimeren werden vor allem lösliche bzw. an Blutplättchen oder Leukocyten gebundene Proteasen verantwortlich gemacht. (Mannucci et al. Blood 83, 3018-3027 (1994)).

Zur Vermeidung von Problemen, die mit der Herstellung von pdvWF in Zusammenhang stehen, wurde bereits die Herstellung von rekombinantem vWF (rvWF) durch Fermentation von rekombinanten Zellen vorgeschlagen. Fischer et al. (FEBS Letters 351, 345-348 (1994)) beschreiben die Expression des vWF Wildtyps in CHO-Zellen. Die cDNA für vWF wurde in der EP-0 197 592 beschrieben. Der hergestellte rvWF wurde auf seine Molekularstruktur untersucht. Eine gelelektrophoretische Untersuchung hat gezeigt, dass die Multimerenstruktur des rvWF mit der idealen Struktur des pdvWF übereinstimmt, wobei die hochmolekularen Strukturen ebenfalls nachgewiesen werden konnten. Jedoch wurde gefunden, dass der rvWF nicht in die Triplett-Struktur aufgetrennt wird, welche für den pdvWF charakteristisch ist. Diese Triplett-Struktur enthält eine prominente Bande und zwei oder mehr Satellitenbanden, welche in einem Komplex mit der prominenten Bande des pdvWF auftreten.

Die grosstechnische Herstellung von rvWF wird von Schlokat et al. (Thrombosis and Haemostasis 73, Abstr. 993 (1995)) beschrieben. Der in CHO-Zellen hergestellte rvWF wurde chromatographisch gereinigt, wobei keine Reduktion der spezifischen Ristocetin-Kofaktoraktivität erfolgte. Durch die Co-expression des Enzyms Furin wurde die Prozessierung des Pro-Proteins zum reifen rvWF erreicht. Die erhaltene Ristocetin-Kofaktoraktivität war für alle untersuchten Klone relativ hoch, z. B. 70 Einheiten pro Einheit vWF-Antigen.

Die Ristocetin-Kofaktoraktivität des vWF wird üblicherweise gemäss einer Vorschrift von Weiss et al. (Journal of Clinical Investigation 52, 2708-2716 (1973)) bestimmt. Dabei wird die Aktivität des vWF als Cofaktor für die Ristocetin induzierte Aggregation von Blutplättchen untersucht.

Diese Aktivität dient als Mass für die Wirksamkeit des vWF zur Behandlung der vW-Krankheit. Von Fricke et al. (Thrombosis and Haemostasis 70, 351-356 (1993)) wurde in einem Plasmastandard der World Health Organisation (WHO) für Faktor VIII und vWF ein sehr hohes Verhältnis von 0,92 Ristocetin-Kofaktoraktivität (RCoF) zu vWF-Antigen (vWF:Ag) gefunden.

Die im Stand der Technik beschriebenen vWF-Präparate enthalten den vWF in proteolytisch abgebauter Form. Die Stabilität dieser Präparate ist dadurch begrenzt. Auch die Versuche zur Verhinderung der Proteolyse nach Abnahme einer Blutprobe in Gegenwart von entsprechenden Inhibitoren führten nicht zu einem vWF mit intakter Struktur.

Alle vWF-Konzentrate, die durch Reinigung des Proteins aus humanem Blutplasma erhalten werden oder in Kontakt mit biologischem Material aus Säugetieren getreten sind, sind ausserdem potentiell mit dem Risiko behaftet, krankheitserregende Moleküle, wie z.B. Viren, vom Plasmaspender zu enthalten.

Ein weiteres Problem für die vWF Präparate des Stand der Technik liegt in der geringen Halbwertszeit des vWF nach Verabreichung an einen Patienten . In diesem Zusammenhang musste auch eine verringerte Halbwertszeit des FVIII:C festgestellt werden. Die Halbwertszeit für exogenen FVIII:C liegt generell unter der von endogenen FVIII:C und ist im Fall von plasmatischen FVIII:C üblicherweise ca. 10 h, im Fall von rekombinantem FVIII:C nicht mehr als ca. 12 h.

So liegt die Halbwertszeit für ein FVIII:C/vWF Konzentrat von CENTRE REGIONAL DE TRANSFUSION SANGUINE DE LILLE (FRANCE) gemäss Produktbeschreibung 1992 bei 10 bis 14 h; nach Gabe dieses Präparates konnte ein Anstieg des endogenen FVIII:C auf lediglich 1 - 3 Einheiten pro Milliliter in 24 h beobachtet werden. Für ein anderes FVIII:C/vWF enthaltendes Präparat (Haemate® HS Behring) wird die Halbwertszeit mit 7 Stunden bei Typ 3 vW-Krankheit, 12,3 h bei Typ 2A und 13,8 h bei Typ 1 angegeben.

Die Erfindung stellt sich zur Aufgabe, das Indikationsspektrum für den vWF zu erweitern und eine verbesserte Pharmakokinetik von Faktor VIII in vivo zu erreichen.

Die Aufgabe wird durch den Gegenstand der Patentansprüche gelöst.

Es wurde überraschenderweise gefunden, dass FVIII:C in vivo besonders gut und lang andauernd stabilisiert werden kann, wenn von einem vWF mit der hohen Halbwertszeit von mehr als 20 h, vorzugsweise mindestens 30 h, eingesetzt wird. Als wirksame Komponente kann nicht nur nativer, humaner vWF bereitgestellt werden, sondern auch funktionell aktive Analoge, Derivate, Fragmente und Mutanten. Vorzugsweise wird rekombinanter vWF (rvWF) erfindungsgemäss eingesetzt. Es ist weiters bevorzugt, eine Fraktion des vWF in der erfindungsgemässen Präparation zur Verfügung zu stellen. Der vWF kann durch chromatographische Reinigungsverfahren, insbesondere durch Ionenaustausch und/oder Affinitätschromatographie fraktioniert werden, wobei die Fraktionen gewonnen werden können, die eine hohe Halbwertszeit bzw. anhaltende und persistente Erhöhung der Faktor VIII-Spiegels in vivo zeigen.

Es hat sich überraschenderweise herausgestellt, dass durch die Verabreichung eines vWF mit verlängerter Halbwertszeit der FVIII:C nicht nur stabilisiert wird, sondern FVIII:C in nachhaltiger Weise endogen induziert wird. Die Induktion des körpereigenen FVIII:C spielt vor allem dann eine Rolle, wenn der Organismus nicht mit exogenem FVIII:C belastet werden soll. Im Fall einer vW-Krankheit wird oftmals gleichzeitig ein Mangel an FVIII:C gefunden, obwohl der Patient nicht an Hämophilie A leidet (Pseudohämophilie). Die Verabreichung des FVIII:C wäre daher nicht unbedingt erforderlich, falls durch die Verabreichung des vWF genügend endogener FVIII:C produziert wird.

Eine Art der Pseudohämophilie ist zurückzuführen auf Mutationen im vWF-Molekül im Bereich zwischen 1 und 272, die die Bindung des FVIII:C beeinträchtigen. Im Fall von vWF Normandi ist beispielsweise eine Punktmutation für die fehlende Bindungskapazität für FVIII:C verantwortlich.

Dementsprechend eignet sich ein vWF mit einer verlängerten Halbwertszeit zur erfindungsgemässen Anwendung an einen Patienten mit angeborenen oder erworbenen Mangel an funktionellem vWF. Erfindungsgemäss werden insbesondere die folgenden Krankheiten bzw. durch die Krankheiten verursachte Zustände gemäss der Klassifikation von Sadler et al., Thromb. Haemostasis 74:161-166 (1995) behandelt. Darunter finden sich alle vW-Krankheiten, die auf Mutationen des vWF-Moleküls zurückzuführen sind, aber auch die vW-Krankheiten vom Typ 1, 2, 3, die durch einen Mangel an vWF gekennzeichnet sind. Diese Krankheiten sind unter den Begriff der funktionellen Störungen des vWF zu subsumieren.

In einer speziellen Ausführungsform werden Krankheitszustände behandelt, die im Zusammenhang mit dem Auftreten von Antikörpern gegen vWF bzw. FVIII:C stehen. Eine weitere bevorzugte Ausführungsform betrifft die Behandlung von Patienten, die an phänotypischer Hämophilie A leiden.

Die Behandlung kann einerseits prophylaktisch und andererseits therapeutisch vorgenommen werden. Ein Präparat enthaltend vWF mit einer verlängerten Halbwertszeit eignet sich insbesondere für die Prophylaxe, z.B. vor einer möglichen Verletzung des Patienten oder vor einer Operation, welche mehrere Stunden dauert.

Die Verabreichung des vWF kann dabei je nach Art der Krankheit mit oder ohne Verabreichung von FVIII:C kombiniert werden. Ein FVIII:C Gehalt in der pharmazeutischen Präparation ist vor allem dann wünschenswert, wenn eine akute Blutung rasch gestillt werden soll. Da die Erhöhung des endogenen FVIII:C Gehalts im Blut des Patienten erst nach einigen Stunden relevant wird, ist eine initiale Verabreichung von FVIII:C gemeinsam mit dem vWF vorzuziehen.

Die Bereitstellung einer pharmazeutischen Präparation enthaltend FVIII:C und vWF zur erfindungsgemässen Anwendung hat den Vorteil, dass der FVIII:C nicht nur in vivo stabilisiert wird, sondern auch in vitro. Die Kombination mit FVIII:C als natives Protein oder ein entsprechendes funktionelles Mittel, ein Derivat, ein Analog oder eine Mutante ist besonders vorteilhaft, besonders im Fall von rvVF. Die Stabilität des eingesetzten Präparates ist vor allem für Infusionspräparate relevant, welche über eine Dauer von mehreren Stunden einen Patienten infundiert werden können ohne Risiko der Veränderung des Präparates und deren Notwendigkeit der Veränderung des Dosierschemas.

Es hat sich überraschenderweise herausgestellt, dass der vWF mit der verlängerten Halbwertszeit die Pharmakokinetischen Eigenschaften des FVIII:C wesentlich verbessert. So kann erfindungsgemäss auch ein Präparat zur Verfügung gestellt werden, welches den biologisch aktiven FVIII:C/vWF-Komplex enthält, dessen verbesserte pharmakokinetischen Eigenschaften, wie eine hohe in vivo - "recovery" (Wiederfindung oder biologische Verfügbarkeit) bzw. eine verlängerte Halbwertszeit in vivo für den FVIII:C v.a. zurückzuführen sind auf den Gehalt an vWF mit einer verlängerten Halbwertszeit. Das erfindungsgemässe FVIII:C/vWF-Präparat zeigt im allgemeinen eine biologische Halbwertszeit des FVIII:C von mehr als 13 h insbesondere mehr als 17 h, wobei auch Halbwertszeiten von mehr als 25 h möglich sind. Die in vivo - "recovery" des erfindungsgemässen Präparates ist üblicherweise im Bereich von 90 bis 100 %, im Plasma.

Der FVIII:C Anstieg nach Gabe des vWF mit verlängerter Halbwertszeit kann bis auf 6 Einheiten pro Milliliter oder mehr herbeigeführt werden, während im Kontrollversuch bestätigt werden konnte, dass durch Gabe von Haemate® HS Behring im Tierversuch lediglich 3 Einheiten pro Milliliter erreicht worden sind.

Für die erfindungsgemässe Anwendung eignet sich vor allem die mature Form des vWF, welcher zum Grossteil proteolytisch prozessiert ist. Vorzugsweise wird der rvWF verwendet, der durch Kontakt mit einer Protease, wie z.B. Furin oder einer Protease vom Subtilisin-Typ, zumindest 80%, am meisten bevorzugt mehr als 90% bis zu 100%, proteolytisch prozessiert ist.

Erfindungsgemäss wird der vWF in der Art und Konzentration verabreicht, dass der FVIII:C über einen Zeitraum von mindestens 48 Stunden, vorzugsweise mehr als 72 Stunden in vivo stabilisiert wird. Der FVIII:C Gehalt in vivo gilt dann als stabil, wenn trotz Elimination von endogenen bzw. exogenen FVIII:C zumindest 0,1, vorzugsweise mehr als 0,3, Units pro Milliliter Blut enthalten ist. Dieser Mindestgehalt an FVIII:C gewährleistet einen minimalen Schutz des Patienten vor unerwünschten Blutungen.

Entsprechend der lang andauernden und persistenten Wirkung des vWF in vivo kann die initiale Dosis verringert werden, dass das pharmazeutische Präparat erfindungsgemäss ohne Nebenwirkungsreaktionen, wie Thrombosebildung, Thrombozytenaktivierung oder Thrombozytopänie an einem Patienten verabreicht werden kann.

Die Verabreichung der Präparation kann mehrmals erfolgen, wobei durch die hohe Halbwertszeit des vWF längere Intervalle möglich sind, beispielsweise Intervalle von mehr als 12 Stunden, vorzugsweise etwa 24 Stunden (tägliche Verabreichung). Die effektive vWF Menge in der erfindungsgemäss verwendeten pharmazeutischen Präparation führt vorzugsweise zu einer Konzentration an vWF, beispielsweise gemessen als Antigen mit einem entsprechenden ELISA, von mehr als 2 Einheiten/ml Blut über einen Zeitraum von mehr als 24 Stunden, vorzugsweise mehr als 48 Stunden. Die effektive Menge an vWF soll weiters die Blutungscharakteristik normalisieren, d. h. die Blutungsintensität soll deutlich reduziert werden und ein Blutungsstillstand in kürzester Zeit nach einer Verletzung, insbesondere in weniger als 15 Minuten, vorzugsweise weniger als 10 Minuten, herbeigeführt werden.

Um die Übertragung von möglicherweise vorhandenen Viren zu verhindern, ist darauf zu achten, dass vorzugsweise pharmazeutische Präparationen zur Anwendungen kommen, die zur Inaktivierung bzw. Abreicherung von Viren behandelt sind.

Zur Inaktivierung von Viren eignet sich insbesondere eine Hitzebehandlung in Lösung bzw. in festem Zustand, welche sowohl lipidumhüllte als auch nicht-lipidumhüllte Viren verlässlich inaktivieren kann. Beispielsweise wird die erfindungsgemässe Präparation gemäss der EP 0 159 311 in festem, nassen Zustand hitzebehandelt. Andere Verfahren zur Virus-Inaktivierung umfassen auch die Behandlung mit Detergenzien oder chaotropen Substanzen, beispielsweise gemäss der EP 0 519 901, WO 94/13329, DE 44 34 538, EP 0 131 740 und WO 90/15613.

Der vWF wird vorzugsweise als hochreines Protein eingesetzt, welches durch ein chromatographisches Reinigungsverfahren erhalten wird. Die chromatographische Reinigung erfolgt insbesondere durch Ionenaustauschchromatographie und/oder Affinitätschromatographie. Dafür können u. a. Materialien zum Anionenaustausch, darunter synthetische Trägermaterialien oder Träger auf Kohlenhydratbasis, mit Liganden, wie DEAE-, TMAE-, QAE-, Q- oder Aminoalkylgruppen herangezogen werden, bzw. Träger mit immobilisierten Substanzen, die eine spezifische Affinität für vWF aufweisen. Geeignete Affinitätsmaterialien enthalten beispielsweise Heparin.

Dieses Reinigungsverfahren eignet sich v. a. für die grosstechnische Gewinnung des rvWF.

Dabei ist zu beachten, dass der vWF in der erfindungsgemässen Präparation eine ausreichende Resistenz gegenüber einem proteolytischen Abbau aufweist, dass auf den Zusatz üblicher Stabilisatoren verzichtet werden kann. In Ausnahmefällen kann aber auch während des Herstellungsverfahrens ein entsprechender Proteaseinhibitor zugesetzt werden, um die intakte Struktur zu erhalten. Zur weiteren Unterstützung der Stabilität des vWF kann das pharmazeutische Präparat auch ein Polyalkylenglycol, wie PEG oder Polypropylenglycol, oder Glycerin in einer Konzentration enthalten, die den vWF nicht präzipitiert und physiologisch verträglich ist.

Die Formulierung der erfindungsgemäss angewandten Präparationen kann in an sich bekannter und üblicher Weise erfolgen, z.B. mit Hilfe von Salzen und gegebenenfalls Aminosäuren, aber auch in Gegenwart von Tensiden vorgenommen werden. Vorzugsweise werden Salze, wie z.B. Natriumchlorid oder Calziumchlorid, verwendet und ein pH im Bereich von 6-8 gewählt. Als Aminosäuren sind Glycin oder Lysin bevorzugt. Ebenso kann ein pharmazeutisch akzeptabler Puffer gewählt werden. Aufgrund der hohen Stabilität des vWF kann üblicherweise auf die Verwendung von Stabilisatoren, wie Trägerproteine oder Inhibitoren in der Formulierung verzichtet werden.

Vorzugsweise kommt ein rvWF in einem Präparat zur Anwendung, welcher nach elektrophoretischer Analyse Multimerenbanden in Abwesenheit von Satellitenbanden zeigt. Dies entspricht der Struktur eines vWF als nicht-fragmentiertes, also intaktes Protein. Vorzugsweise zeigt der rvWF im pharmazeutischen Präparat das gesamte Spektrum an Multimeren, insbesondere auch den vWF mit hohem Molekulargewicht, ähnlich der nativen Multimerenverteilung.

Eine weitere bevorzugte Präparation enthält jedoch eine vWF-Fraktion, welche vorwiegend aus niedermolekularem vWF besteht. Es hat sich gezeigt, daß ein derartiger "low molecular weight" vWF, welcher nicht das gesamte Spektrum an Multimeren aufweist, in der erfindungsgemäßen Präparation relevant ist. Dekamere und kleinere Oligomere, insbesondere eine Mischung von Di- bis Hexameren, sind bevorzugt. Eine derartige vWF-Fraktion kann durch Auftrennung einer vWF-enthaltenden Lösung nach dem Molekulargewicht bzw. nach der Affinität zu verschiedenen Chromatographiematerialien, die eine Trennung nach dem Molekulargewicht ermöglichen, erhalten werden. Beispielsweise wird die niedermolekulare vWF-Fraktion gemäß der EP 0 705 846 durch affinitätschromatographische Auftrennung eines rvWF an immobiliesiertem Heparin erhalten. Ebenso ist es möglich funktionell defekte Multimere entsprechend dem niedermolekularen Produkt von Wagner et al., The Journal of Cell Biology 101, 112-120 (1985), erfindungsgemäß einzusetzen. Die Verwendung einer vWF-Fraktion mit einem Molekulargewicht von weniger als 5 Mio. Dalton, vorzugsweise weniger als 3 Mio. Dalton, in einer erfindungsgemäßen Präparation hat den Vorteil, daß die Stabilisierung des Faktor VIII in vivo möglich ist, ohne im wesentlichen die Plättchenaggregation und damit hämostatische bzw. thrombotische Prozesse zu beeinflussen, Eine derartige Präparation eignet sich vor allem zur persistenten Stabilisierung des Faktor VIII in vivo in einem Patienten, der nicht mit einer zusätzlichen vWF-Aktivität bzw. einer Plättchen aggregierenden Aktivität belastet werden soll. Eine besonders bevorzugte Ausführungsform bezieht sich auf die Verwendung einer vWF-Fraktion, die praktisch keine Plättchenbindungsaktivität aufweist, entsprechend einem Verhältnis von weniger als 5 Einheiten Ristocetin Cofaktoraktivität pro 100 Einheiten Antigen, vorzugsweise weniger als 3 Einheiten pro 100 Einheit.

Beispielsweise konnte gezeigt werden, daß eine niedermolkulare rvWF-Fraktion in einem vWF-defizienten Hund eine Verdopplung des Faktor VIII-Spiegels über einen Zeitraum von mindestens 48 Stunden erreichte, während keine Änderung vWF-Plasmaaktivität verzeichnet werden konnte.

Gemäss einer bevorzugten Ausführungsform wird der rvWF in einer Form erhalten, die nach Verabreichung an ein Säugetier das Multimerenmuster mit einer Singulettstruktur beibehält. Eine proteolytische Aufspaltung der Singuletts in Satellitenbanden bleibt somit aus.

Die bevorzugte Konzentration des vWF in der verabreichungsfertigen Lösung ist im Bereich von 1 bis 100 Einheiten/ml. Durch die hohe Reinheit des Präparates kann dieses auch in Konzentrationen bis zu 1000 E/ml formuliert werden. Die Aktivität ist durch die Ristocetin-mediierte Plättchenaggregation charakterisiert und wird als Ristocetin-Cofaktor-Aktivität (RCoF) angegeben (siehe dazu Journal of Clinical Investigation 52, 2708-2716, 1973). Die übliche Dosis für den vWF liegt im Bereich von 40-80 RCoF-Einheiten/kg in Intervallen von 6-48 Stunden. Als initiale Dosis kann auch eine höhere Dosis bis zu 200 RCoF gewählt werden.

In einer speziellen Ausführungsform enthält die erfindungsgemäss verwendete pharmazeutische Präparation den vWF als einzigen wesentlichen Bestandteil. Somit kann diese Präparation im wesentlichen aus hochgereinigtem vWF bestehen.

Zur Herstellung der pharmazeutischen Präparationen kann der vWF aus Plasma oder einer Plasmafraktion gereinigt werden, wobei je nach Verwendungszweck die gleichzeitige Anreicherung oder Abreicherung des FVIII:C zweckmässig ist. Wenn von einem Zellkulturüberstand ausgegangen wird kann der vWF ebenfalls in Anwesenheit oder Abwesenheit von FVIII:C gereinigt und aufkonzentriert werden. FVIII:C kann in diesem Fall entweder dem Zellkulturmedien zugesetzt werden bzw. von der Zelle co-exprimiert werden.

Gemäss der Erfindung wird weiters ein diagnostisches Set zur Bestimmung einer phänotypischen Hämophilie A zur Verfügung gestellt, welches
a) vWF in einer pharmazeutisch akzeptablen Form und
b) ein Mittel zur Bestimmung der FVIII:C-Aktivität in einer Blutprobe des Patienten
enthält. Vorzugsweise wird ein vWF mit einer verlängerten Halbwertszeit eingesetzt, insbesondere vWF mit einer Halbwertszeit von mehr als 20 Stunden. rvWF ist für diesen Zweck besonders gut geeignet, da er frei von FVIII:C-Aktivität zur Verfügung gestellt werden kann. Bei einem menschlichen Patienten mit phänotypischer Hämophilie A verursacht die Verabreichung eines humanen vWF Präparates den Anstieg des endogenen FVIII:C Gehaltes. Dieser Anstieg ist um so signifikanter, wenn der vWF den FVIII:C in vivo erfindungsgemäss stabilisiert. Falls der Patient jedoch an einem Defekt der Hemostase leidet, der einen Mangel an FVIII:C verursacht, wie, Hämophilie A, oder falls der Patient Antikörper gegen FVIII:C enthält, die einen Mangel an funktionell aktiven FVIII:C verursachen, bleibt der Anstieg des endogenen FVIII:C aus. Somit kann der vWF mit der verlängerten Halbwertszeit zur Herstellung einer diagnostischen Präparation verwendet werden, die die Differenzierung zwischen einer vW-Krankheit und einer vW-Krankheit mit einem gleichzeitigen Auftreten eines genomischen Defekts mit der Folge eines FVIII:C Mangels, insbesondere Hämophilie A, ermöglicht.

Das Mittel enthält vorzugsweise ein Reagens, welches einen Aktivator der Blutgerinnung und ein Mittel zur Detektion der Blutgerinnung umfaßt.

Das Mittel zur Bestimmung der FVIII:C Aktivität ist entweder ein Mittel zur Bestimmung der Blutgerinnungsaktivität, wobei die Blutgerinnungszeit gemessen wird (Einstufentest oder Zweistufentest), oder aber ein chromogener Test bzw. ein Test zur Bestimmung von aktivem Blutgerinnungsenzymen, die in Abhängigkeit von der FVIII:C Aktivität einer Probe wirken. Vorzugsweise wird ein chromogener Test zur Bestimmung der FVIII:C Aktivität in einer Plasma- oder Serumprobe gemäss der EP 0 496 723 durchgeführt. Als chromogenes Substrat wird in diesem Fall ein Substrat für Faktor Xa eingesetzt.

Es ist aber auch möglich die FVIII:C Aktivität mit Hilfe der aPTT (activated partial thromboplastin time) unter Verwendung eines Faktor VIII Mangelplasmas zu bestimmen. Die aPPT ist ein Beispiel für einen Test, welcher die Aktivierung der Blutgerinnung mit einem entsprechenden Aktivator vorsieht, worauf das Ausmass der Blutgerinnung mit einem entsprechenden Mittel detektiert wird. Das Ausmass der Blutgerinnung kann beispielsweise durch die Erfassung der Blutgerinnungszeit (Endpunktbestimmung) oder der Kinetik der Blutgerinnung detektiert werden.

Die Erfindung wird weiters durch die nachfolgenden, die Erfindung nicht limitierende Beispiele näher beschrieben.

### ABBILDUNGEN

### Abbildung 1

Verlauf der Gerinnungsparameter in einem von Willebrand-defizienten Schwein nach Behandlung mit dem erfindungsgemässen vWF-Präparat.

### Abbildung 2

Verlauf der Gerinnungsparameter in einem von Willebrand-defizienten Schwein nach Behandlung mit einem plasmatischem vWF/FVIII-Konzentrat.

### Abbildung 3

Behandlungsschema einer von Willebrand Faktor-immundepletierten Maus.

### Abbildung 4

Blutungscharakteristik von von Willebrand Faktor-defizienten immundepletierten Mäusen nach Behandlung mit plasmatischem von Willebrand Faktor und dem erfindungsgemässen Präparat mit je 100 E RCoF/kg Körpergewicht im Vergleich zu normalen Mäusen und unbehandelten von Willebrand Faktor-defizienten immundepletierten Mäusen.

### Abbildung 5

Behandlungsschema einer von Willebrand Faktor-immundepletierten Maus zu Beispiel 6.

### Abbildung 6

Blutungsverhalten von von Willebrand Faktor-defizienten immundepletierten Mäusen nach Behandlung mit rekombinantem von Willebrand Faktor und rekombinantem Faktor VIII im Vergleich zu normalen Mäusen und unbehandelten von Willebrand Faktor-defizienten immundepletierten Mäusen.

### Abbildung 7

Wirkung von LMW-rvWF in einem Hund mit vWF-Mangel.

### BEISPIEL 1

### Herstellung eines rekombinanten von Willebrand Faktors aus CHO-Zellen

Ein CHO-Zellklon, der rekombinanten von Willebrand Faktor produziert, wird wie in Fischer *et al*., *FEBS*.*Lett*. 351:345-348, 1994, beschrieben, hergestellt. Durch Transfektion mit einem für die cDNA humanen Furins kodierenden Vektor (van den Ouweland *et al*., *Nucleic Acids Res*. 18:664, 1990) wurde die Zellinie zur Co-Expression von humanem Furin gebracht. Derartige stabile Zellklone wurden im Grossmassstab in Perfusionsreaktoren auf *Microcarriern* fermentiert (Blüml *et al*., In: Spier RE, Griffith JB, Berthold W, eds. Animal cell technology. Oxford, London: Butterworth-Heinemann, 1994:267-269).

Die Reinigung wurde durch ein 2-stufiges chromatographisches Verfahren gemäss *Thromb*.*Haemost*. 73:1160, 1995, durchgeführt. Die durch Elution mit Kochsalz desorbierte Fraktion wurde gewonnen und durch Gelfiltration über Sephadex G25 (Pharmacia) in einen Puffer, enthaltend 5 g/l Na₃Citrat.2H₂O, 2 g/l NaCl, 5 g/l Glycin, 5 g/l L-Lysin.HCl und 0,62 g/l CaCl₂.2H₂O, pH 7,0, umgepuffert. Die Präparation war charakterisiert durch einen von Willebrand Faktor-Antigengehalt von 36,4 E/ml, gemessen in einem kommerziellen von Willebrand Faktor-ELISA (Boehringer Mannheim) und durch eine von Willebrand Faktor-Aktivität von 13,8 E/ml, gemessen mittels Ristocetin-mediierter Plättchenaggregation (Evans *et al*., *Brit*.*J*.*Haematol*. 37:289-294, 1977).

### BEISPIEL 2

### in vivo-Aktivität und Pharmakokinetik von rekombinantem von Willebrand Faktor in einem homozygoten von Willebrand Faktor-defizienten Schwein im Vergleich zu porcinem von Willebrand Faktor.

Für die Untersuchung können von Willebrand-defiziente Tiere, wie z.B. die von Roussi *et al*., *Brit*.*J*.*Haematol*. 90:661-668 1995, beschriebenen homozygoten von Willebrand-defizienten Schweine, verwendet werden. In diesem Versuch wurde ein 4 Monate altes, 37 kg schweres, weibliches, homozygotes von Willebrand-defizientes Schwein eingesetzt. Dieses war charakterisiert durch eine Blutungszeit von über 30 Minuten, gemessen nach der Ohrblutungsmethode von Samama *et al*., *Thromb*.*Haemostas*. 71:663-669, 1994, und einen von Willebrand Faktor-Plasmaspiegel von unter der Nachweisgrenze, sowohl im Antigen-ELISA als auch in der Ristocetincofaktoraktivität bestimmt. Die Faktor VIII-Aktivität betrug ca. 1 E/ml, gemessen als humaner Faktor VIII im 1-Stufen-Gerinnungstest (Immuno), 2-Stufen-Gerinnungstest (Immuno), oder chromogenem Faktor VIII-Test (Immunochrom FVIII:C, Immuno).

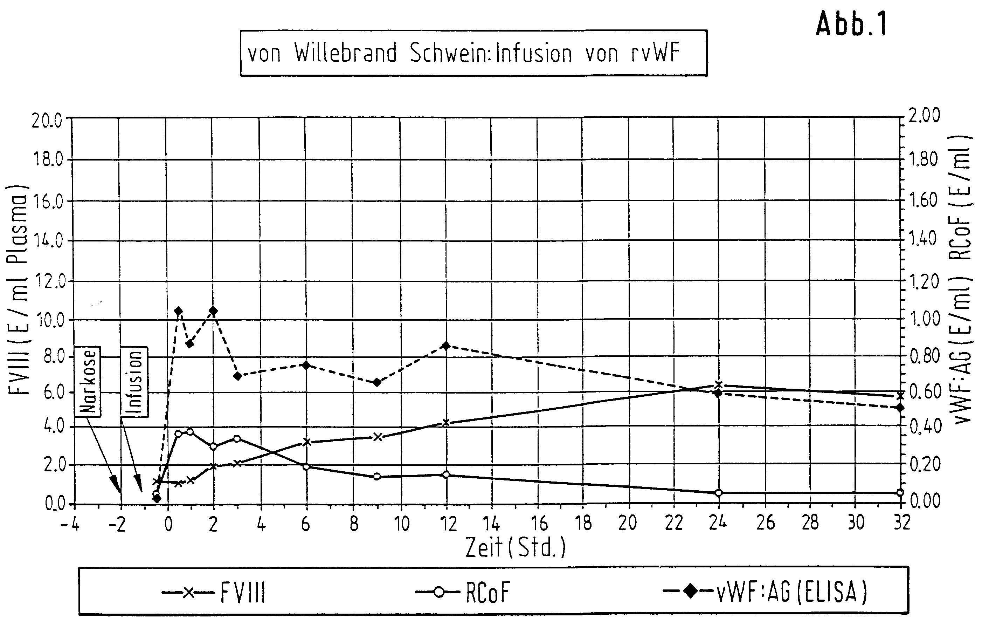

Unter Narkose wurde dem Schwein eine erfindungsgemässe Präparation, die, wie in Beispiel 1 beschrieben, gewonnen wurde, in einer Dosis von 34 RCoF E/kg Körpergewicht injiziert. Unmittelbar vor der Infusion sowie 30 min, 1 Std, 2 Std, 3 Std, 6 Std, 9 Std, 12 Std, 24 Std und 32 Std nach Infusion wurden Blutproben genommen, daraus Citratplasma hergestellt und aus diesem von Willebrand Faktor-Antigen, Ristocetincofaktoraktivität und Faktor VIII nach den oben genannten drei Testmethoden bestimmt. Die Resultate sind Abbildung 1 zu entnehmen. Es zeigte sich, dass nach etwa 24 Stunden, zu einem Zeitpunkt als keine Ristocetincofaktoraktivität des infudierten Präparates *ex vivo* mehr nachweisbar war, eine nachhaltige Verfünffachung des Plasmaspiegels an Faktor VIII eingetreten war. Für das vWF-Antigen konnte eine biologische Halbwertszeit von 32,4 Stunden errechnet werden.

Als Kontrolle wurde ein porcines, plasmatisches von Willebrand Faktor-Präparat unmittelbar nach Entnahme der letzten Blutprobe demselben Schwein in einer Dosis von 45 RCoF E/kg Körpergewicht gegeben. Ebenso wie nach Gabe des erfindungsgemässen Präparates wurden zum Zeitpunkt 30 min, 1 Std, 2 Std, 3 Std, 6 Std, 9 Std, 12 Std, 24 Std und 32 Std nach Infusion Blutproben gezogen, aus diesen Plasma hergestellt und die entsprechenden Faktor VIII/von Willebrand Faktor-spezifischen Gerinnungsparameter bestimmt. Es zeigte sich, dass es auch hier zu einer Induktion des Faktor VIII kam, der jedoch bezogen auf den Ausgangswert nach ca. 10 Stunden aber nur den doppelten Wert erreichte und auch schneller wieder abnahm. Der porcine, plasmatische von Willebrand Faktor zeigte eine Halbwertszeit von ca. 10 Stunden. Als makroskopischen Parameter für die Normalisierung des im defizienten Tier gestörten Gerinnungssystems wurde die Blutungszeit bestimmt, die von über 30 Minuten vor Infusion des von Willebrand Faktors auf ca. 13 Minuten nach Infusion korrigiert werden konnte, wobei dieser Effekt auch noch 32 Stunden nach der Infusion nachweisbar war. Die Infusion des plasmatischen von Willebrand Faktor führte ebenso zu einer Korrektur der Blutungszeit auf ca. 15 Minuten während die Blutungszeit 24 Stunden nach der Infusion des plasmatischen von Willebrand Faktor-Präparates bereits wieder dem Ausgangswert entsprach, also keine Verkürzung feststellbar war.

### BEISPIEL 3

### Pharmakokinetik der Multimere von rekombinantem von Willebrand Faktor im Schwein.

Aus den Plasmaproben des Versuches aus Beispiel 2 wurde die Struktur der von Willebrand Faktor-Multimere durch SDS-Agarosegelelektrophorese im 2 %igen Agarosegel nach der Methode von Ruggeri *et al*., *Blood* 57:1140-1143, bestimmt. Dabei wurden die von Willebrand Faktor-Multimere durch eine immunenzymatische Färbung nach Aihara *et al*., *Thromb*.*Haemostas*. 55:263-267, 1986, sichtbar gemacht. Als primärer Antikörper wurde ein Kaninchen-anti-von Willebrand Faktor-Antiserum (Dakopatts, Glostrup, Denmark) in einer Verdünnung von 1:5000 verwendet. Als sekundärer Antikörper diente ein alkalische Phosphatase-konjugierter, affinitätsgereinigter Ziegen-anti-Kaninchen-IgG H + L Antikörper (Axell, Accurate Chemical and Scientific Corp., NY) in einer Verdünnung von 1:1000. Die Färbung der Proteinbanden erfolgte mittels des Nitroblau-tetrazolium-chloridbrom-indolyl-phosphatsubstratsystems.

Vor Behandlung mit dem erfindungsgemässen Präparat konnte kein von Willebrand Faktor im Schwein nachgewiesen werden. Nach Gabe des Präparates zeigte sich, eine für den nativen Zustand atypische Struktur eines aus Singuletts bestehenden Multimerenmusters, welches auf einen nicht proteolytisch verdauten von Willebrand Faktor zurückzuführen ist. Diese Struktureigenschaft blieb über den gesamten Beobachtungszeitraum unverändert, d.h. keine proteolytische Degradation des Präparates fand statt. Das Präparat wurde allerdings entsprechend der Pharmakokinetik aus der Zirkulation sukzessive eliminiert. Multimere des niedrigsten Molekulargewichtes blieben bis zu 32 Stunden nach Infusion des Präparates nachweisbar.

### BEISPIEL 4

### In vivo-Aktivität und Pharmakokinetik eines plasmatischen von Willebrand/Faktor VIII-Konzentrates in einem homozygoten von Willebrand Faktor-defizienten Schwein.

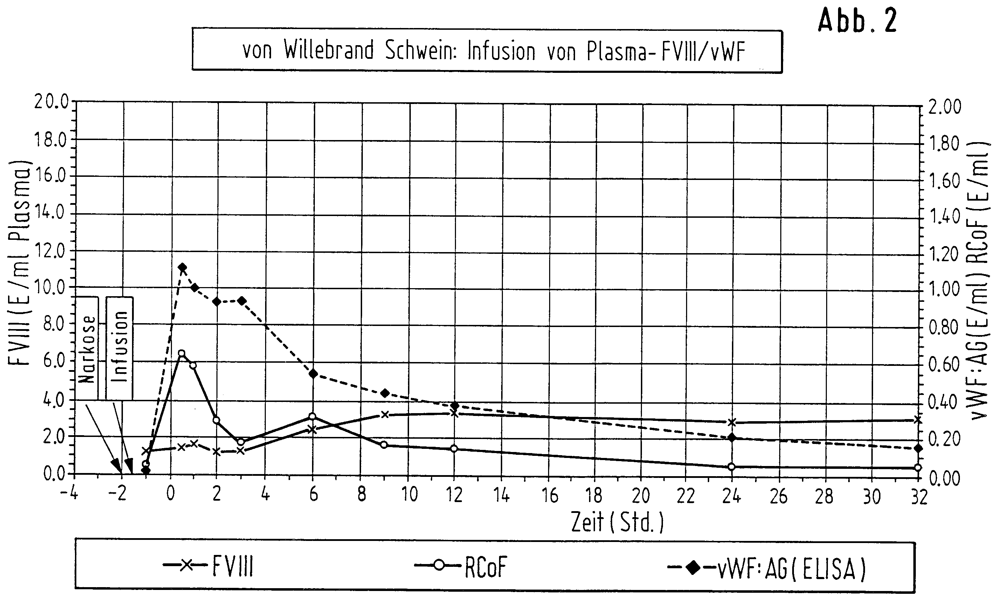

Analog zum Beispiel 2 wurde einem 91 kg schweren weiblichen, homozygoten, vWF-defizienten Schwein ein humanes, plasmatisches FVIII/vWF-Konzentrat, HAEMATE HS 1000 (Fa. Behring), in einer Dosierung von 34 RCoF E/kg KG gegeben. Dieses Präparat ist ein Faktor VIII- und von Willebrand Faktor-enthaltendes Konzentrat. Entsprechend wurde mit der Gabe des von Willebrand Faktors dem Schwein eine Dosis von 17 IE FVIII/kg KG verabreicht. Unmittelbar vor der Infusion sowie 30 min, 1 Std, 2 Std, 3 Std, 6 Std, 9 Std, 12 Std, 24 Std und 32 Std nach der Infusion wurden Blutproben genommen, Citratplasma hergestellt und aus diesem von Willebrand Faktor-Antigen, Ristocetincofaktoraktivität und Faktor VIII nach den im Beispiel 2 beschriebenen Testmethoden bestimmt. Die Resultate sind Abbildung 2 zu entnehmen. Es zeigte sich, dass etwa 12 Stunden nach der Infusion des Präparates, zu einem Zeitpunkt, wo noch ein messbarer Plasmaspiegel an von Willebrand Faktor-Ristocetincofaktoraktivität nachweisbar war, die FVIII-Plasmakonzentration auf das 2,8-fache angestiegen war. Durch externe Zuführung von Faktor VIII stieg der FVIII-Spiegel unmittelbar nach der Infusion um ca. 0,4 E/ml Plasma an. Dies entsprach einer theoretischen Wiederfindungsrate von 94 % des infundierten Faktor VIII.

Für das vWF-Antigen wurde eine biologische Halbwertszeit von 7,5 Stunden errechnet. Damit zeigte sich eine deutlich kürzere biologische Halbwertszeit des humanen, plasmatischen von Willebrand Faktors im Vergleich zum rekombinanten von Willebrand Faktor aus Beispiel 2.

Analog zu Beispiel 3 wurde die Multimerenstruktur des von Willebrand Faktors in den Schweineplasmaproben analysiert. Die schnellere Elimination aus der Zirkulation als die des rekombinanten von Willebrand Faktors konnte auch hier nachgewiesen werden und war mit den in der Abbildung 2 angegebenen Antigenkonzentrationen vergleichbar. Das Präparat zeigte die für von Willebrand Faktor typische Triplett-Struktur, die auf eine proteolytische Degradation zurückzuführen ist.

### BEISPIEL 5

### Vergleich der in vivo-Aktivität eines plasmatischen von Willebrand Faktor/Faktor VIII-Konzentrates und eines rekombinanten von Willebrand Faktors in einer temporär von Willebrand Faktor-defizienten Maus.

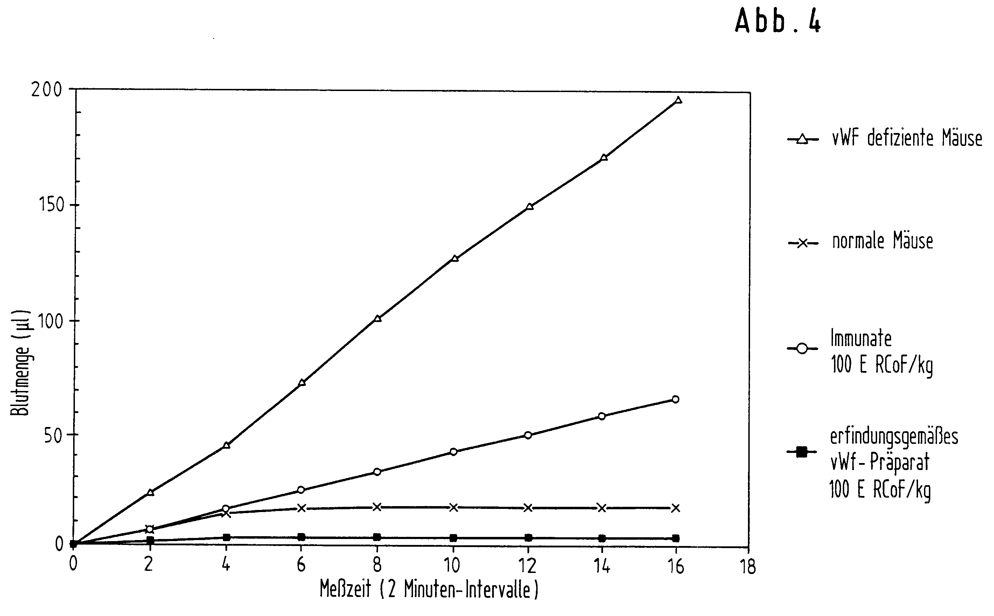

Weibliche NMRI-Mäuse (ca. 20 - 30 g) werden verwendet und durch Gabe von 65 mg/kg Pentobarbital narkotisiert. Anschließend wird die Blutungscharakteristik mit einer modifizierten Methode nach Novak *et al*., *Brit*.*J*.*Haematol*. 69:371-378, 1988, durch definierte Verletzung der Schwanzspitze bestimmt. Das aus der arterio-venösen Verletzung der Schwanzspitze austretende Blut wird auf Filtern (Pipetman P5000-Schutzfilter, Gilson) so gesammelt, daß das Blut direkt den Filter tropfen kann, ohne von diesem durch Kapillarwirkung aufgesogen zu werden, um zu verhindern, daß ein sich formierendes Blutgerinnsel zerstört wird. Die Filtereinheiten werden alle 2 Minuten gewechselt und das austretende Blut in Fraktionen gesammelt. Die Blutsammlung wird 16 Minuten fortgesetzt. Danach wird, soferne die Blutung nicht zum Stillstand gekommen ist, die Wunde verödet. Die Qualifizierung der Blutungscharakteristik erfolgt durch Extraktion des in Fraktionen auf den Filtern gesammelten Blutes jeweils mit 5 ml 0,04 %iger Ammoniumhydroxidlösung über 5 Stunden. Dabei lysieren die Erythrozyten, die mit dem Blut im Filter gesammelt sind. Durch eine 10-minütige Ultraschallbehandlung (Sonorex RK100, Bandelin electronic, Berlin) wird das Haemoglobin extrahiert und quantitativ photometrisch bei 416 nm gegen eine Eichkurve bestimmt. Eine solche kann ermittelt werden indem Mausblutvolumina zwischen 10 µl - 1 ml auf die Filter pipettiert werden, diese wie oben beschrieben extrahiert werden und das entsprechende Haemoglobin photometrisch bei 416 nm bestimmt wird. Entsprechend lassen sich lineare Eichkurven erstellen, die eine direkte Umrechnung der Haemoglobinkonzentration auf die Blutmenge pro Filter ermöglichen. Die Blutungscharakteristik wird ermittelt, indem graphisch die einzelnen Blutfraktionen additiv gegen die Zeit aufgetragen werden. Der Blutungsverlauf einer gesunden Maus ist unter diesen Bedingungen dadurch charakterisiert, daß in einem Beobachtungszeitraum zwischen 6 min und Versuchsende kein weiteres Blut aus der künstlich herbeigeführten Verletzung austritt, also die Blutung bereits zuvor zum Stillstand gekommen ist. In der Graphik zeichnet sich ein derartiger Verlauf durch Parallelität zur x-Achse aus (siehe Abbildung 4).

Mäuse werden nun mit einem anti-von Willebrand Faktor-Inhibitor in einer Dosis von 10 ml/kg Körpergewicht vorbehandelt. Das anti-von Willebrand Faktor-Inhibitorplasma wird durch Immunisierung einer ca. 50 kg schweren 1,5 Jahre alten Ziege mit einem von Willebrand Faktor-Präparat wie folgt gewonnen:
1 g Kryopräzipitat wird in 35 ml einer Tris/Lysin-gepufferten NaCl-Lösung (85 mM) gelöst. Proteine des Prothrombinkomplexes werden durch Adsorption an AlOH₃ und BaSO₄ entfernt. Dazu wird die von Willebrand Faktor/Faktor VIII enthaltende Lösung mit 0,1 Gew.-% AlOH₃ 1 Stunde bei 22 °C inkubiert.
Anschließend wird der AlOH₃-Proteinkomplex durch Zentrifugation abgetrennt. Der gewonnene Überstand wird weiters mit 0,1 Gew.-% BaSO₄ unter Rühren inkubiert und der BaSO₄-Proteinkomplex neuerlich durch Zentrifugation abgetrennt. Der Überstand wird anschließend auf pH 6,8 eingestellt und durch Chromatographie über Q-Sepharose® FF (Pharmacia) gereinigt. Der von Willebrand Faktor-Komplex wird an das in einer Säule gepackte Gel adsorbiert; nicht-bindende Proteine werden durch Waschen mit 4 Säulenvolumina des Startpuffers entfernt. Die Elution der Proteinfraktion wird mit einem Puffer (50 mM Tris/HCl, 500 mM NaCl, pH 6,8) durchgeführt. Das gewonnene Eluat wird mit 50 mM Tris/HCl-Puffer, pH 6,8, auf eine Konzentration von 175 mM NaCl verdünnt und anschließend durch Gelfiltration auf Sepharose® CL6B (Pharmacia) weiter gereinigt. Die Gelfiltration wird mit einem linearen Fluß von 5,7 cm/h in einer Säule mit 26 mm Durchmesser und einer Betthöhe von 82 cm durchgeführt. Die Proteinfraktion des Ausschlußvolumens wird bei 280 nm UV-spektroskopisch detektiert. Der Protein-Peak des Ausschlußvolumens wird durch Ultrafiltration, mit einem Ultrafilter mit einer Retentionsmasse von 30.000 D konzentriert. Ein so hergestellter von Willebrand Faktor/Faktor VIII-Komplex hat eine spezifische Aktivität von 173 Ristocetin-Cofaktoreinheiten (E RCoF) vWF/mg Protein (bestimmt mittels Proteinbestimmung nach Bradford, Anal.Biochem. 72:248-254, 1976). Die Präparation ist frei an Gerinnungs-faktoren II, VII, IX, X, XI und XII.

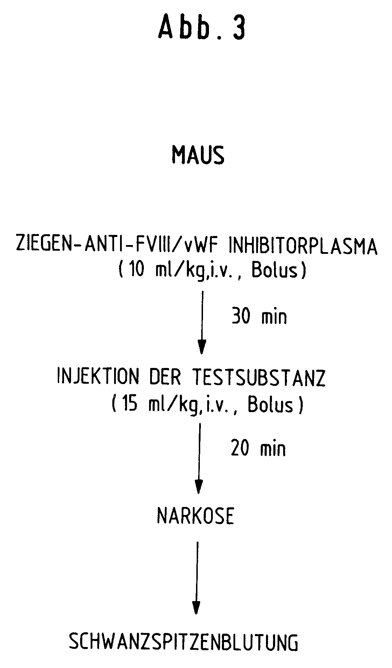

Mäuse werden nun mit dem anti-von Willebrand Faktor-Inhibtiorplasma nach dem Schema aus Abbildung 3 vorbehandelt. Eine Maus mit derart indizierter temporärer von Willebrand-Defizienz zeigt bei Infusion von physiologischer Kochsalzlösung in der nachfolgenden Schwanzspitzenblutungscharakteristik eine stetige Blutung vom Beginn des Meßzeitraumes bis zum Abschluß des Experimentes (siehe Abbildung 4). In diesem Modell des von Willebrand/Jürgens-Syndroms werden nun ein Präparat, enthaltend plasmatischen von Willebrand Faktor, sowie ein erfindungsgemäßes Präparat auf ihre Wirkung untersucht. Dabei werden in beiden Fällen 100 E vWF Ristocetincofaktoraktivität (RCoF)/kg jeweils als Bolus gegeben. Die Blutungscharakteristik ist der Abbildung 4 zu entnehmen. Überraschenderweise zeigt sich, daß ein gemäß Beispiel 1 hergestelltes von Willebrand Faktor-Präparat in diesem Ansatz die Blutungscharakteristik der von Willebrand-defizienten Maus auf die einer gesunden Maus korrigiert, während das plasmatischen von Willebrand Faktor-Präparat (IMMUNATE® STIM4, Immuno) in derselben Dosierung zwar eine deutliche Reduktion der Blutungsintensität bewirkt, aber zu keinem Blutungsstillstand wie das erfindungsgemäße Präparat führt.

Nach Gabe des rekombinanten von Willebrand Faktor-Präparates und durchgeführter Bestimmung der Blutungs-charakteristik wurde den Tieren eine Blutprobe durch Herzpunktion entnommen. Aus *ex vivo* Plasmaproben wurde die Mulitmerenzusammensetzung des infundierten vWF bestimmt. Es zeigte sich, daß die Singulettstruktur auch nach Infusion über den gesamten Meßzeitraum erhalten blieb.

### BEISPIEL 6

### Pharmazeutisches Präparat enthaltend rekombinanten vWF/FVIII-Komplex in einer temporär von Willebrand Faktor-defizienten Maus.

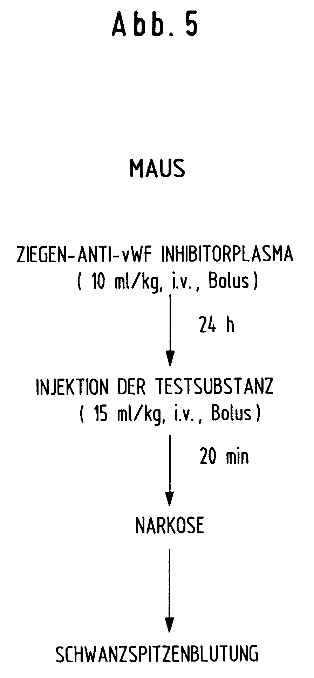

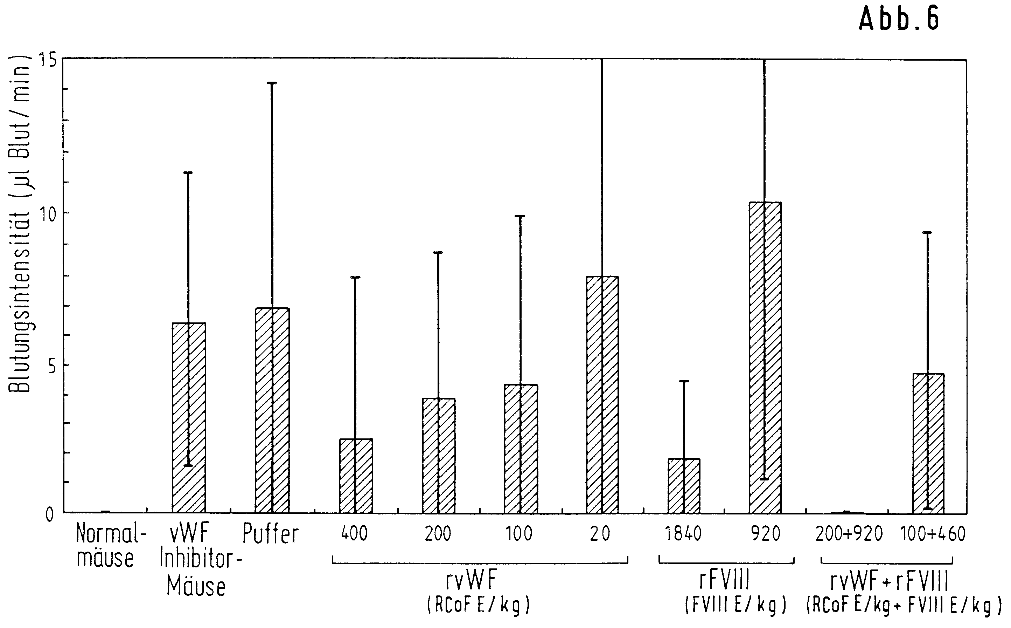

Weibliche NMRI-Mäuse, ca. 20 - 30 g, werden mit einem anti-vWF-Inhibitorplasma, welches durch Immunisierung einer Ziege mit einem rekombinanten vWF gewonnen wurde, analog zu Beispiel 5 behandelt. Das aus der Immunisierung resultierende vWF-Antiplasma reagiert mit Maus-vWF in vitro und in vivo kreuz. Bei Gabe eines anti-vWF enthaltenden Antiplasmas in einer Dosis von 10 ml/kg als i.v. Bolusinjektion kommt es bereits eine Stunde nach Verabreichung zu einer deutlichen Reduktion des vWF-Antigenplasmaspiegels in der Maus, jedoch ist die FVIII-Plasmakonzentration im Gegensatz zu einer Behandlung mit einem anti-vWF-Inhibitor aus Beispiel 5 erst auf ca. 50 % des Normalwertes reduziert. Da das verwendete Antiplasma keine Antikörper gegen FVIII enthält, ist dieser Effekt ein sekundärer der vWF-Immundepletion. Werden Mäuse mit demselben anti-vWF-Inhibitorplasma behandelt und anschließend eine Wartezeit von 24 Stunden eingehalten, ist der vWF-Plasmaspiegel unter die Nachweisgrenze abgesunken und als sekundärer Effekt der induzierten vWF-Defizienz auch die FVIII-Plasmakonzentration auf ≤10 % des Normalwertes abgesunken. Verbunden damit kommt es zu einer signifikanten Steigerung der Blutungsneigung, welche wie in Beispiel 5 durch die Schwanzblutungscharakteristik gemessen wird. In Gruppen zu jeweils 10 Tieren wurden nun derart mit anti-vWF-Antiplasma vorbehandelte Mäuse nach einer Wartezeit von 24 Stunden entweder mit Puffer als Kontrolle oder mit rvWF in steigenden Dosen von 20, 100, 200 und 400 RCoF E/kg behandelt (siehe Behandlungsschema, Abbildung 5). Ebenso wurden Mäuse mit rFVIII (Recombinate, Baxter) in Dosen zu 920 und 1840 E/kg behandelt und Mäuse mit einer pharmazeutischen Zusammensetzung enthaltend rvWF und rFVIII in Dosen von 100 vWF RCoF E und 460 FVIII E/kg sowie 200 vWF RCoF E in Kombination mit 920 FVIII E/kg behandelt. Die Blutungscharakteristik aller Mäuse im Verhältnis zu gesunden Mäusen wurde, wie in Beispiel 5 beschrieben, bestimmt; die Ergebnisse sind in Abbildung 6 zusammengefaßt.

Die Balken geben die Blutungsintensität wieder, die im Rahmen des Schwanzspitzenblutungsexperiments gemessen wird, wobei die Blutungsintensität als die Steigung der Blutungscharakteristik-kurven, wie sie in Abbildung 4 dargestellt sind, im Zeitintervall zwischen 6 und 16 min definiert wird. Rekombinanter vWF führt in steigenden Dosen zu einer deutlichen Reduktion der Blutungsintensität. In diesem Mausmodell kommt es allerdings im Gegensatz zu dem in Beispiel 5 beschriebenen zu keiner Normalisierung des Blutungsverhaltens. Rekombinanter FVIII führt mit steigender Dosis ebenso zu einer deutlichen Reduktion des Blutungsverhaltens, jedoch zu keiner vollständigen Normalisierung des Blutungsverhaltens. Erst die Kombination von rvWF mit rFVIII in einer Dosis von 200 vWF RCoF E und 920 FVIII E/kg führt zu einer vollständigen Normalisierung des abnorm gesteigerten Blutungsverhaltens.

### Beispiel 7

### vWF in einem Hund mit schwerer von Willebrand-Krankheit

Eine Fraktion enthaltend rekombinanten niedermolekularen von Willebrand Faktor (LMW-rvWF) wurde durch Chromatographie an immobilisiertem Heparin gemäß der Vorschrift aus EP 0 705 846 gewonnen. rvWF aus Beispiel 1 wurde als Ausgangsmaterial herangezogen. Der erhaltene LMW-rvWF wurde in einem Puffer enthaltend 5 g/l Natriumcitrat.2H₂O, 2 g/l NaCl, 5 g/l Glyzin, 5 g/l l-Lysinhydrochlorid, 0,62 g/l CaCl₂.2H₂O, pH 7,0 formuliert. Diese Präparation hatte einen Gehalt an vWF-Antigen von 200 E/ml und eine sehr geringe Ristocetin Cofaktor-Aktivität von 3 E/ml. Die Untersuchung der Multimeren nach Elektrophorese auf einem 2%-igen Agarose SDS-Gel zeigte, daß die Präparation im wesentlichen Di-, Tetra- und Hexamere der vWF-Monomere enthielt.

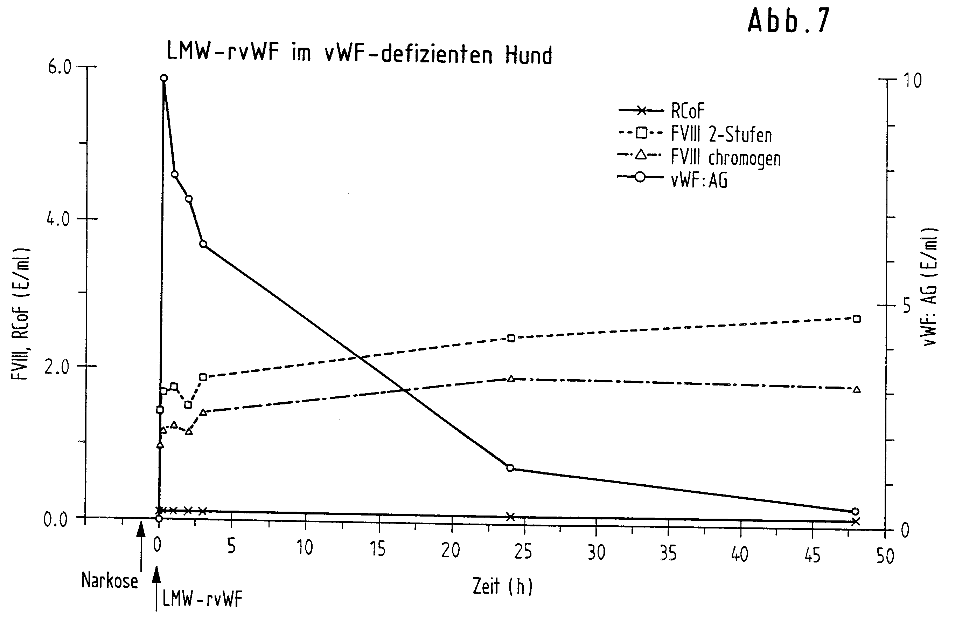

Ein weiblicher Hund von 9 kg Körpergewicht, welcher einen vWF-Mangel zeigte, hatte einen vWF-Antigengehalt in Plasma von weniger als die Nachweisgrenze und eine Faktor VIII-Aktivität von 50% des Normalwertes. Dieses Tier wurde anästhesiert und erhielt eine Infusion der LMW-rvWF Präparation in einer Dosis von 450 Einheiten/kg Körpergewicht entsprechend einem Verhältnis von 45 mg vWF-Antigen/kg Körpergewicht. Vor der Infusion und 15 Minuten, 30 Minuten, 1 Stunde, 2 Stunden, 3 Stunden, 25 Stunden und 48 Stunden nach der Infusion des niedermolekularen vWF wurden Plasmaproben genommen und darin vWF Antigen, Ristocetin Cofaktor-Aktivität und Faktor VIII Aktivität, gemessen im 2-Stufentest und nach einem chromogenen Bestimmungsverfahren, analysiert. Die Resultate werden in Abbildung 7 gezeigt. Die Plasmaproben wurden auch mit einer Agarose-Gelelektrophorese analysiert, wie in Beispiel 3 beschrieben, und die vWF-Oligomere über den Beobachtungszeitraum aufgenommen.

Zuerst verschwanden die hochmolekularen Formen bevor die vWF Oligomere aus dem Blutkreislauf eliminert wurden. In Abbildung 7 wird gezeigt, daß die Faktor VIII-Aktivität erhöht war und 24 Stunden nach der Infusion etwa 200% des Ausgangswertes erreichte, wobei dieser Wert mindestens über die darauf folgenden 24 Stunden stabil blieb. Gleichzeitig nahm jedoch vWF-Antigen schrittweise ab, wobei vWF-Antigen und die Oligomere vollständig aus der Zirkulation verschwanden. Während des Beobachtungszeitraumes konnte in den Plasmaproben keine vWF-Aktivität ausgedrückt durch Ristocetin Cofaktor-Einheiten gemessen werden.

Die Resultate bewiesen, daß ein vWF eine Faktor VIII-Aktivität in Plasma stabilisieren und induzieren kann und somit die intrinsische Gerinnung fördern kann, obwohl dieser keine Plättchenbindungsaktivität aufweist.

## Patentansprüche

1. Pharmazeutische Präparation zur anhaltenden Erhöhung des Blutgerinnungsfaktor VIII-Spiegels in einem Säugetier, enthaltend von Willebrand-Faktor (vWF) bzw. eine vWF-Fraktion mit einer verlängerten biologischen Halbwertszeit.

2. Präparation nach Anspruch 1, dadurch gekennzeichnet, dass der vWF bzw. die vWF-Fraktion eine in vivo Halbwertszeit von mehr als 20 Stunden aufweist.

3. Präparation nach einem der Ansprüche 1 bis 2, dadurch gekennzeichnet, dass der vWF bzw. die vWF-Fraktion eine aus Singuletts bestehende Multimerenstruktur zeigt.

4. Präparation nach einem der Ansprüche 1 bis 3, dadurch gekennzeichnet, dass der vWF bzw. die vWF-Fraktion das gesamte Spektrum an Multimeren, insbesondere auch solche mit hohem Molekulargewicht, ähnlich der nativen Multimerenverteilung zeigt.

5. Präparation nach einem der Ansprüche 1 bis 3, dadurch gekennzeichnet, daß die vWF-Fraktion im wesentlichen aus niedermolekularem vWF mit einem Molekulargewicht von weniger als 5 Mio. Dalton, vorzugsweise weniger als 3 Mio. Dalton, besteht.

6. Präparation nach einem der Ansprüche 1 bis 5, dadurch gekennzeichnet, dass die verabreichungsfertige Lösung den vWF bzw. die vWF-Fraktion in einer Konzentration im Bereich von 1 ist 1000 E/ml enthält.

7. Präparation nach einem der Ansprüche 1 bis 6, dadurch gekennzeichnet, dass der vWF bzw. die vWF-Fraktion chromatographisch gereinigt ist.

8. Präparation nach einem der Ansprüche 1 bis 7, dadurch gekennzeichnet, dass rekombinanter vWF (rvWF) bzw. eine Fraktion des rvWF enthalten ist.

9. Präparation nach einem der Ansprüche 1 bis 8, dadurch gekennzeichnet, dass rvWF bzw. die rvWF-Fraktion durch Kontakt mit einer Protease zumindestens 80%, vorzugsweise mehr als 90% bis zu 100%, proteolytisch prozessiert ist.

10. Pharmazeutische Zusammensetzung, enthaltend die Präparation nach einem der Ansprüche 1 bis 9, wobei der vWF bzw. die vWF-Fraktion mit Faktor VIII komplexiert ist.

11. Pharmazeutische Zusammensetzung nach Anspruch 10, dadurch gekennzeichnet, dass der Faktor VIII biologisch aktiv und ein natives Protein bzw. ein funktionelles Agens, eine Mutante oder ein Derivat ist.

12. Pharmazeutische Zusammensetzung nach Anspruch 10 bis 11, dadurch gekennzeichnet, daß der Faktor VIII eine in vivo Halbwertszeit von mehr als 13 Stunden, vorzugsweise mehr als 17 Stunden aufweist.

13. Verwendung einer Präparation nach Anspruch 1 bis 9 zur Herstellung einer pharmazeutischen Zusammensetzung nach einem der Ansprüche 10 bis 12.

14. Verwendung eines von Willebrand Faktor (vWF) bzw. einer vWF-Fraktion mit einer verlängerten biologischen Halbwertszeit zur Herstellung einer Präparation nach Anspruch 1 zur Stabilisierung von Blutgerinnungsfaktor VIII (FVIII:C) in einem Säugetier.

15. Verwendung nach Anspruch 14, dadurch gekennzeichnet, dass der vWF bzw. die vWF-Fraktion eine in vivo Halbwertszeit von mehr als 20 Stunden aufweist.

16. Verwendung nach Anspruch 14 oder 15, dadurch gekennzeichnet, dass die Präparation zur Erhöhung des endogenen Gehalts an FVIII:C geeignet ist.

17. Verwendung nach einem der Ansprüche 14 bis 16, dadurch gekennzeichnet, dass die Präparation an einen Patienten mit angeborenem oder erworbenem Mangel an funktionellem vWF verabreicht wird.

18. Verwendung nach Anspruch 17, dadurch gekennzeichnet, dass die Präparation an einen Patienten verabreicht wird, der an funktionellen Störungen des vWF, insbesondere von Willebrand-Krankheit leidet.

19. Verwendung nach Anspruch 17, dadurch gekennzeichnet, dass die Präparation an einen Patienten verabreicht wird, der Antikörper gegen den vWF bzw. FVIII:C aufweist.

20. Verwendung nach Anspruch 17, dadurch gekennzeichnet, dass die Präparation an einen Patienten verabreicht wird, der an phänotypischer Hämophilie A leidet.

21. Verwendung nach einem der Ansprüche 14 bis 20, dadurch gekennzeichnet, dass die Präparation rekombinanten vWF (rvWF) bzw. eine Fraktion des rvWF enthält.

22. Verwendung nach Anspruch 21, dadurch gekennzeichnet, dass der rvWF bzw. die rvWF-Fraktion durch Kontakt mit einer Protease zumindestens 80%, vorzugsweise mehr als 90% bis zu 100%, proteolytisch prozessiert ist.

23. Verwendung nach einem der Ansprüche 14 bis 22, dadurch gekennzeichnet, dass die Präparation in Intervallen von mehr als 12 Stunden, vorzugsweise täglich, wiederholt an das Säugetier verabreicht wird.

24. Verwendung nach einem der Ansprüche 14 bis 23, dadurch gekennzeichnet, dass die Präparation in Kombination mit einem FVIII:C-Präparat verabreicht wird.

25. Verwendung nach einem der Ansprüche 14 bis 24, dadurch gekennzeichnet, dass die Präparation zusätzlich FVIII:C insbesondere rekombinanten FVIII:C enthält.

26. Verwendung nach einem der Ansprüche 14 bis 25, dadurch gekennzeichnet, dass die Präparation zur Stabilisierung des FVIII:C-Gehaltes über einen Zeitraum von mindestens 48 Stunden geeignet ist.

27. Verwendung von vWF bzw. einer vWF-Fraktion zur Herstellung einer Präparation zur Bestimmung einer phänotypischen Hämophilie A.

28. Verwendung nach Anspruch 27, dadurch gekennzeichnet, dass die Präparation vWF bzw. eine vWF-Fraktion mit einer Halbwertszeit von mindestens 20 Stunden, insbesondere rvWF, enthält.

29. Verwendung nach Anspruch 27 oder 28, dadurch gekennzeichnet, dass differenziert wird zwischen einer von Willebrand-Krankheit und einer Krankheit mit einem gleichzeitigen Auftreten eines genomischen Defekts mit der Folge eines FVIII:C-Mangels, insbesondere Hämophilie A.

30. Diagnostisches Set zur Bestimmung einer phänotypischen Hämophilie A, enthaltend
a) vWF bzw. eine vWF-Fraktion in einer pharmazeutisch akzeptablen Form und
b) ein Mittel zur Bestimmung des FVIII:C-Aktivität in einer Blutprobe des Patienten.

31. Diagnostisches Set nach Anspruch 30, dadurch gekennzeichnet, dass der vWF bzw. die vWF-Fraktion frei von FVIII:C-Aktivität ist.

32. Diagnostisches Set nach Anspruch 30 oder 31, dadurch gekennzeichnet, dass der vWF bzw. die vWF-Fraktion mit einer Halbwertszeit von mindestens 20 Stunden, insbesondere rvWF, enthalten ist.

33. Diagnostisches Set nach einem der Ansprüche 30 bis 32, dadurch gekennzeichnet, dass das Mittel zur Bestimmung der FVIII:C-Aktivität ein Reagenz umfasst, welches einen Aktivator der Blutgerinnung und ein Mittel zur Detektion der Blutgerinnung enthält.

34. Diagnostisches Set nach Anspruch 33, dadurch gekennzeichnet, dass das Mittel zur Detektion der Blutgerinnung ein chromogenes Substrat enthält, welches spezifisch für den Faktor Xa ist.
